# EUROPEAN PATENT APPLICATION

(11) **EP 3 797 811 A1**
(43) Date of publication of application: **31.03.2021**
(21) Application number: 19199736.0
(22) Date of filing: 26.09.2019
(51) Int. Cl.: A61M 5/315, A61M 5/142, A61M 5/168, A61M 5/20

(54) **DEVICE FOR EXPELLING A FLUID MEDICAMENT DRIVEN BY AN ELECTRO-ACTIVE POLYMER ACTUATOR**

(71) Applicant: Ypsomed AG, 3401 Burgdorf (CH)
(72) Inventor: Bosshard, Simon, 3006 Bern (CH); Steck, Jürg, 3422 Kirchberg (CH)

(57) **Abstract**

Device for delivering fluid medicament comprising a reservoir with the medicament having
a piston closing the reservoir moveable in a delivery direction towards an outlet of the reservoir. The device having a gear rack for moving the piston comprising a series of serrated teeth. Retaining means and driving means each engage a tooth of the series and each engagement prevents movement of the gear rack opposite to the delivery direction and allows movement in the delivery direction. The retaining means is secured with respect to the reservoir such that when the driving means is set in a reciprocating motion into and opposite the delivery direction, then the driving means slaves the gear rack to move together with the driving means into the delivery direction. The device is characterized by an electro-active polymer (EAP) actuator stacked between the retaining and driving means setting the driving means in the reciprocating motion.

## Description

### FIELD OF THE INVENTION

The current invention relates to a device for delivering a fluid medicament using a linear drive mechanism using an electroactive polymer (EAP) actuator setting a driving means and/or retaining means in a reciprocating motion to advance a piston in a reservoir containing the fluid medicament.

### BACKGROUND OF THE INVENTION

Fluid medicaments may be delivered to patients from a reservoir either via intravenous or subcutaneous injection. A piston or plunger in the reservoir is advanced by a delivery mechanism towards an outlet of the reservoir such that medicament located between the piston and the outlet can be injected. There are several ways to advance the piston, either using a pumping system, for example an infusion pump. Typically, a rotational movement of an electromotor is converted into a linear movement of a piston rod which itself abuts or is connected to the piston in the reservoir. An example is presented in US20050197626, where a telescopic piston rod arrangement is advanced by an electromotor using a gear box between the piston rod and the motor. This requires additional parts such as gear wheels and occupies space.

Alternatively, medicament is delivered using an injection pen whereby a linear movement of a dose setting member is converted in an advancing movement of the piston rod. The linear movement is provided by a thumb or finger of a patient pushing on the dose setting member which may be directly transmitted into a linear advancement of the piston rod. The piston rod can be shaped as a rack with serrated teeth engaging driving and retaining means which are set in a reciprocating linear movement by the dose setting member during multiple deliveries, as disclosed in US6613023. Such a pen requires a relatively high force that needs to be applied by the user. Alternatively, the linear movement of the patient pushing on the dose setting member is geared down and converted into a rotational movement that itself is used for advancement of the piston rod, see for example US6004297. Although the user needs less force, more parts and space are required for the gearing mechanism.

Electroactive polymers (EAPs) are subjected to dimensional changes when a voltage is applied and are used within medical devices as a fluid flow restrictor, where the dimensions of a passage for a fluid are changed by applying a voltage across the EAP, such as described in WO13097956 or EP2276163. Alternatively, EAPs are used in a series of linear actuators aligned next to each other to form a peristaltic displacement pumping mechanism, see for example WO08157351.

It is an object of the present invention to provide a delivery device for an injection or infusion device that does not rely on the force of the patient to advance the piston rod. Furthermore it is an object to provide a linear advancement mechanism without the need of space consuming transmission gears for back and forth conversion of linear movement into rotational movement.

This object is solved by a delivery device based on a drive mechanism using an electroactive polymer (EAP) actuator which converts electrical energy into a reciprocating linear motion that is used for advancing a gear rack with serrated teeth engaging retaining and driving means that are connected to the EAP actuator. Another solution for the problem is that the reciprocating motion is used for advancing the EAP actuator versus a fixed gear rack.

### DEFINITIONS

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an infusion set, an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example "a gear rack" does not exclude the fact that there may be two gear racks that functionally or structurally fulfill the purpose of "a gear rack". The mere fact that certain elements or steps are recited in distinct claims shall not preclude the existence of further meaningful combinations of these elements or steps.

### DESCRIPTION OF THE INVENTION

In an embodiment according to the invention, a device for delivering or expelling a fluid medicament is presented where a relative motion between a gear rack and an electroactive polymer (EAP) actuator is used for advancing a piston in a reservoir to expel medicament. The device for delivering or expelling the fluid medicament comprises a reservoir with the fluid medicament, the reservoir is preferably enclosed in a housing of the device or in a housing part such as a frame holding the drive mechanism based on the EAP actuator. The piston closes the reservoir and can be moved with respect to the reservoir in a delivery direction towards an outlet of the reservoir for expelling the fluid medicament. The piston is typically a rubber plug engaging a wall surface of the reservoir and the reservoir typically is a glass cartridge with an opening for receiving the rubber plug on one end and having a pierceable septum at the other end. The device further comprises a gear rack having a series of teeth, and retaining means and driving means each engaging a tooth of the series of teeth. The teeth of the series of teeth may be serrated teeth or asymmetric teeth such as saw teeth. Each engagement prevents relative movement between the gear rack and the retaining means or the driving means in one direction and allows relative movement between the gear rack and the retaining means and driving means in the opposite direction. Either the retaining means or the gear rack is secured with respect to the reservoir, preferably with respect to the housing. The driving means can be set in a reciprocating motion into and opposite the delivery direction and the driving means is configured to slave the gear rack or the retaining means to move together with the driving means into the delivery direction such that the gear rack, or the retaining means moves the piston in the reservoir. The device is characterized by an electro-active polymer (EAP) actuator which is stacked between, and fixed to the retaining means and the driving means and which sets the driving means in the reciprocating motion. The reciprocating motion is a back and forth motion with a defined stroke length and during each reciprocating motion the piston in the reservoir is advanced by a distance equal to the stroke length.

The driving means and the retaining means are spaced apart along the delivery direction. Preferably, the pitch of the teeth (i. e. the distance between two adjacent teeth) of the serrated teeth of the gear rack is at least equal to the defined stroke length of the reciprocating motion.

The combination of the gear rack, the retaining means and the driving means together with the reciprocating motion by the EAP actuator, enable a relative linear motion between the gear rack and the retaining/driving means and, hence, this relative linear motion is used for advancement of the piston in the reservoir. The advancement of the piston is based on linear movement without using rotational movement and therefore, no conversion of rotational movement into linear movement is taking place. As a consequence, no gearings, gear boxes or transmitters are required making the system less complex and space occupying. Furthermore, the EAP actuators have the advantage that they can operate within a wide range of frequencies essentially reciprocating motions of up to the kHz frequencies are feasible thus at a high frequency and a low stroke length accurate advancement of the piston can be achieved thereby increasing the accuracy of the delivery device.

The EAP actuator is a multilayered stack of capacitors comprising a dielectric layer and a conductor wherein the layers are oriented perpendicular to the delivery direction and wherein application of an (actuation) voltage across the multilayered stack of capacitors results in a contraction or elongation along the delivery direction and wherein a voltage modulation, such as on/off switching, sets the driving means in the reciprocating motion with a defined stroke length. The dielectric layer is an elastomer film with a defined and constant film thickness, preferably each layer of the stack has a thickness below 50 micrometer, more preferably below 25 micrometer, even more preferably below 15 micrometer. The dielectric layer is made from a dielectric elastomer such as a silicone, an acrylic elastomer or a polyurethane. By playing with the number and shape of the stacked layers, the total length and shape of the EAP actuator can be tuned. Typically the number of layers ranges between 50 and 5000 layers, preferably between 100 and 4000 layers, most preferably between 200 and 2000 layers. The conductor layer is typically based on carbon (e.g. carbon black) and is compliant in a plane perpendicular to the delivery direction to accommodate the dimensional changes of the dielectric layers as an actuation voltage is applied. Using the voltage modulation at different frequencies also results in reciprocating movements at different frequencies making the EAP stack a versatile actuator for the drive mechanism. The actuation voltage applied across the multilayered stack is below 2500V, preferably below 1500 volt and more preferably below 1000V, for example an actuation voltage of around 800V. Depending on the number of layers selected, the layer thickness and the voltage applied, the stroke length can be varied preferably above 0.5mm, more preferably above 1 mm and most preferably above 2.0 mm.

The EAP actuator has end plates at each end of the stack such that a contraction of the EAP stack is effectively transmitted to other parts such as the driving means and retaining means which are preferably directly or indirectly coupled to the end plates. In advantageous embodiments one of the two end plates is operatively coupled to the housing or to the reservoir in the housing.

In advantageous embodiments the EAP actuator has an elongated shape with a disc shaped cross section having a central hole enclosing the gear rack along the delivery direction. In further advantageous embodiments the EAP actuator has a C-shaped cross section and at least partially surrounds the gear rack along the delivery direction. The EAP actuators are produced using printing techniques such as disclosed in EP2891194 giving a high degree of freedom in the cross sectional shape of the EAP actuator. The advantage of selecting an EAP actuator comprising a hole (or channel) is that the EAP actuator at least partially encloses the gear rack. As a consequence a space efficient design can be used for the device. A C-shaped cross section has the additional benefit that the gear rack can be mounted from the side and does not have to be inserted along the axis defined by the delivery direction. Alternatively, the shape of the EAP actuator is adjusted to the housing encapsulating the EAP actuator, or the shape of the EAP actuator is adjusted both to the housing and the gear rack.

Optionally, the multilayer stack of capacitors in the EAP actuator comprises a plurality of passive layers, e. g. not contributing to the contraction of the EAP layer when an actuation voltage is applied. The passive layer being designed as a capacitor, and wherein a sensor voltage is applied across such passive layers during the reciprocating EAP movement, the change of capacitance of the passive layer may be assessed. A change in capacitance measured may be used to measure dimensional changes, for example the changed thickness of the dielectric layers which again can be used to calculate forces upon mechanical loading the EAP actuator. Thus by introducing the passive layers a sensor may be formed for measuring forces when the piston in the reservoir is advanced. Forces will increase when the piston reaches the end of the reservoir and/or during an occlusion in the fluid delivery path resulting in compression of the dielectric layer sandwiched between two passive electrodes. The thickness of the compressed layer is measured and used to calculate the force acting on the EAP. Using passive layers enables detection of, for example, an empty cartridge or an error as a result of an occlusion, therewith increasing the reliability and control options for the medicament delivery device.

In some embodiments the retaining means in the device comprises at least one retaining tooth located at a resilient retaining arm and wherein the retaining tooth forms a one-way retaining ratchet with a tooth of the series of teeth of the gear rack. Alternatively, the retaining means comprises a fixed tooth or protrusion engaging a resilient arm on the gear rack or the series of serrated teeth of the gear rack is resiliently coupled to the housing. Applying more than one retaining tooth has the advantage of an increased the reliability of the device should one of the retaining tooth fail due to fatigue or become brittle due to physical ageing.

The one way retaining ratchet prevents relative movement between the gear rack and the retaining means in one direction and allows relative movement in the opposite direction. For example the gear rack may be moved to advance the piston versus an EAP actuator that is axially fixed relative to the reservoir except for the allowed reciprocating movement. In a specific example the one-way retaining ratchet prevents movement of the gear rack opposite to the delivery direction, preventing undesired backdrive of the gear rack. The retaining means is axially fixed with respect to the reservoir (or housing) and the gear rack is advanced with respect to the reservoir (or housing) by the reciprocating movement of the driving means with respect to the reservoir (or housing).

In some embodiments the driving means comprises at least one driving tooth located at a resilient driving arm and the driving tooth forms a one-way driving ratchet with a tooth of the series of serrated teeth of the gear rack. Alternatively, the driving means comprises a fixed tooth or protrusion engaging a resilient arm on the gear rack or the series of serrated teeth of the gear rack is resiliently coupled to the housing. The one-way driving ratchet is essentially identical to the retaining ratchet in forming a one way ratchet, the driving ratchet is however used to drive the gear rack in the specific example mentioned above for a moving gear rack. The reciprocating movement of the driving means results in a reciprocating release and formation of the retaining one-way ratchet and the driving one-way ratchet. Applying more than one driving tooth has the advantage of an increased the reliability of the device should one of the driving tooth fail due to fatigue or become brittle due to physical ageing.

In some embodiments, each tooth of the series of serrated teeth comprises a surface oriented essentially perpendicular to the delivery direction and a surface that is oblique to the delivery direction. Essentially perpendicular is defined here as having a higher inclination angle with respect to the delivery direction compared to the oblique surface. The surface of the tooth oriented perpendicular to the delivery direction is used for a form-fit (or friction-fit) engagement either for piston advancement using the driving means or the form fit (or friction-fit) engagement is used to prevent backdrive using the retaining means. The oblique surfaces of the serrated teeth provide a ramp that can be used by the retaining means or driving means to flex and jump to the next tooth in the series of serrated teeth.

The retaining tooth and/or the driving tooth comprise a surface that is complementary to the oblique surface of the serrated teeth of the gear rack, and a surface that is complementary to the essentially perpendicular surface of the serrated teeth of the gear rack and wherein a form-fit engagement exists between the essentially perpendicular surfaces of the retaining tooth and/or the driving tooth and the tooth of the gear rack to prevent relative movement between the gear rack and the retaining means and driving means in one direction and wherein a friction fit engagement exists between the oblique surfaces of the retaining tooth and/or the driving tooth and the tooth of the gear rack to allow relative movement between the gear rack and the retaining means and driving means in the opposite direction. In some embodiments the retaining arm of the retaining means and/or the driving arm of the driving means is flexed as the gear rack or the driving means is advanced in the delivery direction. For the flexing, the ramped (oblique) surface of each tooth of the gear rack is used to allow relative movement between the gear rack and the retaining means.

Optionally, the gear rack comprises a plurality of series of serrated teeth that are axially displaced relative to another along the delivery axis and wherein each series of serrated teeth is each engaged with a retaining means and a driving means. By using a plurality, for example two series of serrated teeth, the accuracy of the device can be optimized as the peaks of one series of serrated teeth is exactly positioned between the peaks of the other one series of serrated teeth. Therewith the incremental advancement steps (strokes) for the EAP actuator/gear rack combination can be optimized and therewith the minimal step for advancing the piston in the reservoir. For the device, the series of serrated teeth of the gear rack are positioned along and oriented perpendicular to the delivery direction. Alternatively, the gear rack comprises a plurality of series of serrated teeth that are not axially displaced relative to another along the delivery axis and wherein each series of serrated teeth is each engaged with a retaining means and a driving means.

Optionally, the gear rack can be rotated around its axis to disengage the retaining means and/or the driving means from the gear rack such that relative movement along the delivery direction is allowed in both directions for resetting the device to a starting position. The EAP actuator driven device may be used for a disposable or for a reusable device. For a reusable device, the piston rod has to be reset to the initial starting position to insert a new (and full) reservoir when the previous one has been emptied. As mentioned above, the advancement mechanism is based on one-way ratchet systems preventing backdrive of the gear rack and consequently the one-way ratchets must be disengaged to enable reset. By rotating the gear rack relative to the actuator, the one-way ratchets are disengaged. Preferably, the end plates that are coupled to the EAP actuator are not rotated as the gear rack is rotated and thereby the driving means and/or the retaining means are disengaged from the rotating gear rack, thereby allowing for a reset or backwards movement of the gear rack.

The gear rack or drive means either directly advances the piston or a gear box is located between the gear rack or drive means and the piston. A gear box between the gear rack (or EAP actuator) and the piston in the reservoir may gear up or gear down the linear movement (between gear rack and/or driving means) to either increase the stroke length of the piston (at a lower force) or to increase the force of the piston on the fluid (at a lower stroke length). Thereby the versatility of the device can be increased, for example for delivery of highly viscous medicaments requiring higher delivery forces.

Optionally, the device comprises a second EAP actuator engaging the same series of serrated teeth of the gear rack or another series of teeth from the plurality of series of serrated teeth.

In some embodiments, the device further comprises a housing and wherein the housing or a part that is attached or attachable to the housing holds the reservoir, and wherein the gear rack or the retaining means is directly or indirectly fixed to the housing.

In an embodiment according to the invention, a device for delivering or expelling a fluid medicament is presented where a movement of a gear rack with respect to a reservoir or housing is used for advancing a piston to expel medicament. The gear rack is moved with respect to a driving means that is capable to fulfill a reciprocating motion. The device for delivering or expelling the fluid medicament comprises the reservoir with the fluid medicament, and the reservoir is axially and rotationally fixed with respect to a housing or a housing part of the device. The device comprises a piston closing the reservoir moveable with respect to the reservoir in a delivery direction towards an outlet of the reservoir for administering the fluid medicament. Furthermore a gear rack for moving the piston in the delivery direction with a series of serrated teeth is provided. The device comprises retaining means and driving means each engaging a tooth of the series of serrated teeth and each engagement prevents movement of the gear rack opposite to the delivery direction and allows movement of the gear rack in the delivery direction, and wherein the retaining means is secured with respect to the reservoir. Secured means that the retaining means is rotationally and axially fixed with respect to the reservoir or with the housing of the device when the device is in an operative state. The driving means can be set in a reciprocating motion with respect to the retaining means into and opposite the delivery direction. The reciprocating motion is a linear motion into and contrary to the delivery direction and in one of the two motions the driving means slaves the gear rack to move together with the driving means into the delivery direction. The device is characterized by an electro-active polymer (EAP) actuator stacked between, and fixed to the retaining means and the driving means and which sets the driving means in the reciprocating motion.

The device as just described wherein the retaining means comprises a retaining tooth located at a resilient retaining arm and wherein the retaining tooth forms a one-way retaining ratchet with a tooth of the series of serrated teeth of the gear rack, and wherein the driving means comprises a driving tooth located at a resilient driving arm and wherein the driving tooth forms a one-way driving ratchet with a tooth of the series of serrated teeth of the gear rack.

In a further advantageous arrangement according to the embodiment just described each tooth of the series of serrated teeth comprises a surface oriented essentially perpendicular to the delivery direction and a surface that is oblique to the delivery direction.The retaining tooth and/or the driving tooth comprise a surface that is complementary to the oblique surface of the serrated teeth of the gear rack, and a surface that is complementary to the essentially perpendicular surface of the serrated teeth and wherein a form-fit engagement between the essentially perpendicular surfaces of the retaining tooth and the tooth of the gear rack prevents movement of the gear rack opposite to the delivery direction, and wherein a form-fit engagement between the essentially perpendicular surfaces of the driving tooth and the tooth of the gear rack slaves the gear rack in the delivery direction as the driving means is moved into the delivery direction during the part of the reciprocating movement of the driving means into the delivery direction (no actuation voltage applied) the driving ratchet is in a form fit with the gear rack whereas the retaining ratchet is released. In the subsequent part of the reciprocating motion of the drive means opposite to the delivery direction (actuation voltage applied), the driving ratchet is released whereas the retaining ratchet is in a form fit with the gear rack. As the gear rack is moved with respect to the reservoir (or housing) the retaining arm of the retaining means is flexed as the gear rack is moved in the delivery direction and the complementary oblique surface of the retaining tooth slides over the oblique surface of a tooth to the next tooth of the series of tooth of the gear rack. Thus the one-way retaining ratchet is released as the gear rack is advanced by the engagement of the driving ratchet and reciprocating motion of the driving means. The reciprocating motion being initiated by the voltage modulation of the EAP actuator.

The movements of the driving means opposite to the delivery direction releases the engagement between the driving means and the series of serrated teeth while the engagement between the retaining means and the series of serrated teeth is maintained. The movement of the driving means into the delivery direction releases the engagement between the retaining means and the series of serrated teeth of the gear rack while the gear rack is advanced into the delivery direction. In the embodiment just described the device comprises a housing and wherein the housing or a part that is attached or attachable to the housing holds the reservoir, and wherein the retaining means is directly or indirectly fixed to the housing.

Optionally, the gear rack may be a segmented gear rack whereby each segment can pivot with respect to an adjacent segment, for example using a hinge or film link such that the gear rack can bend in one direction as the EAP actuator advances the segmented gear rack. The gear rack thus can transform from a linear to a bent configuration to reduce the dimensions of the device in the delivery direction

In a further embodiment, a device for delivering or expelling a fluid medicament is presented where a movement of a driving means or a retaining means with respect to a gear rack is used for advancing a piston in a reservoir to expel medicament. In this embodiment, the gear rack is axially fixed with respect to the reservoir or housing as the driving means and retaining means crawl along the gear rack to advance the piston and deliver medicament.

The device for delivering or expelling the fluid medicament of the present embodiment comprises a reservoir with the fluid medicament and a piston closing the reservoir which can be moved in a delivery direction towards an outlet of the reservoir for administering the medicament. The gear rack comprises a series of serrated teeth, wherein the gear rack is secured with respect to the reservoir or housing. Retaining means and driving means each engage a tooth of the series of serrated teeth and the retaining and driving means are spaced apart by the EAP actuator. Each engagement prevents movement of the retaining means and driving means opposite to the delivery direction and allows movement of the retaining means and driving means with respect to the gear rack in the delivery direction. The driving means can be set in a reciprocating motion into and opposite the delivery direction and is configured to slave the retaining means to move together with the driving means into the delivery direction along the gear rack to move the piston. The device is characterized by an electro-active polymer (EAP) actuator stacked between, and fixed to the retaining means and the driving means and which sets the driving means in the reciprocating movement.

The device of the present embodiment wherein further and optionally the retaining means comprises a retaining tooth located at a resilient retaining arm and wherein the retaining tooth forms a one-way retaining ratchet with a tooth of the series of serrated teeth of the gear rack, and wherein the driving means comprises a driving tooth located at a resilient driving arm and wherein the driving tooth forms a one-way driving ratchet with a tooth of the series of serrated teeth of the gear rack.

The retaining tooth and/or the driving tooth comprise a surface that is complementary to the oblique surface of the serrated teeth of the gear rack, and a surface that is complementary to the essentially perpendicular surface of the serrated teeth and wherein a form-fit engagement between the essentially perpendicular surfaces of the retaining tooth and the tooth of the gear rack prevent movement of the retaining means opposite to the delivery direction, and wherein a form-fit engagement between the essentially perpendicular surfaces of the driving tooth and the tooth of the gear rack moves the driving means and the retaining means in the delivery direction.

The device of the present embodiment may further comprise a housing and wherein the housing or a part that is attached or attachable to the housing holds the reservoir, and wherein the gear rack is directly or indirectly fixed to the housing.

### BRIEF DESCRIPTION OF THE DRAWINGS

The subject matter of the invention will be explained in more detail in the following text with reference to preferred exemplary embodiments which are illustrated in the attached drawings, in which:
Figure 1: Schematic overview for a device for delivering or expelling a fluid medicament using an EAP actuator to deliver a fluid medicament from a reservoir,
Figure 2: Overview of a drive system for a device according to a first embodiment with a moving gear rack,
Figure 3: Drive system for a moving gear rack with an EAP actuator stacked between a drive means and a retaining means,
Figure 4: Drive system for a moving gear rack with an EAP actuator stacked between a drive means and a retaining means,
Figure 5: Detail of Figure 3,
Figure 6: Drive system in a reset configuration,
Figure 7: Detail for drive system in a reset configuration,
Figure 8: Drive system for a device according to a second embodiment, the EAP actuator forming a passage for the gear rack,
Figure 9: EAP actuator for a device according to a third embodiment with a C shaped cross section having an opening for receiving a gear rack,
Figure 10: Device for delivering or expelling a fluid medicament according to a fourth embodiment, the gear rack is fixed and the EAP actuator crawls over the gear rack, and
Figure 11: Top view of device displayed in Figure 10.

The reference symbols used in the drawings, and their primary meanings, are listed in summary form in the list of reference signs.

### DETAILED DESCRIPTION

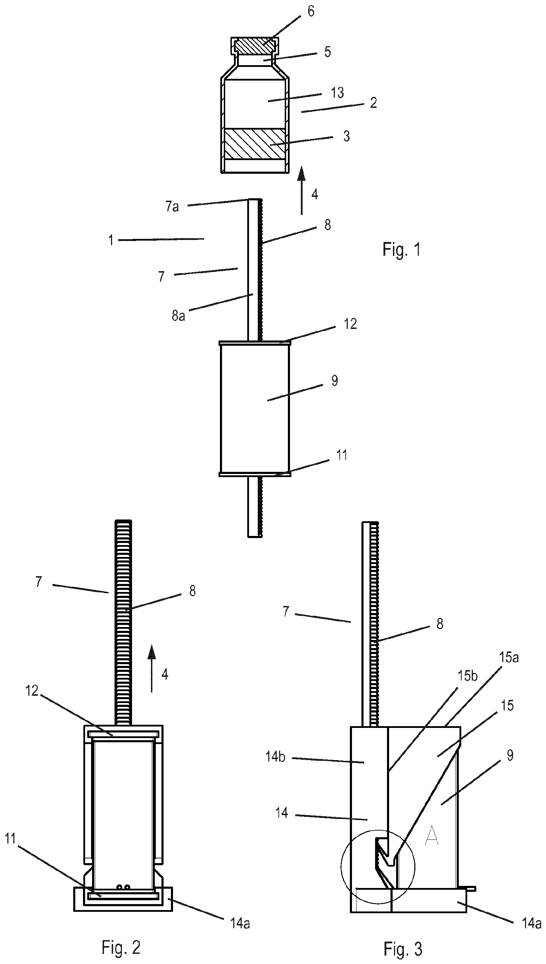

A schematic representation of a device 1 for delivering or expelling a fluid medicament according to the invention is shown in Figure 1. The device comprises a reservoir 2, preferably a glass cartridge closed by a piston 3 having an outlet 5 closed by a piercable septum 6. Alternatively outlet 5 is closed by a luer connector. A liquid medicament 13 is located between the piston 3 and the outlet 5 and the piston 3 can be advanced towards the outlet 5 defining a delivery direction 4. Once a needle or infusion set (not shown) pierces the septum 6 (or a part connected to the luer), the medicament 13 can be delivered from the device 1. The glass cartridge 2 has a cylindrical shape defining a cartridge axis. A gear rack 7 has a longitudinal axis which is aligned with the axis of the cartridge 2. The gear rack 7 may be advanced towards the piston 3 in the cartridge along the delivery direction 4 whereby a distal end 7a of the gear rack 7 abuts and advances the piston 3 or, alternatively a not shown flange is coupled to the gear rack 7 which abuts the piston 3 to evenly distribute the forces across the proximal surface of the piston 3. The gear rack 7 comprises a series of serrated teeth 8 extending from a proximal section to a distal section of the gear rack 7 and the teeth 8 are oriented perpendicular to the axis of the gear rack. The surface 8a opposite to the series of serrated teeth 8 on the gear rack is substantially smooth and lacks any technical geometrical structures. The gear rack 7 has substantially circular cross section, but other shapes such a triangular, squared or octagonal shapes may be used as well. The gear rack 7 is driven by an electroactive polymer (EAP) actuator 9 comprising a stack of electroactive polymer layers, preferably polysiloxane (silicone), and each polymer layer is sandwiched between conductive layers. The conductive layers may be contacted alternating to one of the two electrodes supplying the actuation voltage.

The electroactive polymer layers are made from a dielectric elastomer such as a silicone (for example polydimethylsiloxane, Dow Corning silicone (DC 3481), an acrylic elastomer (for example 3M acrylic elastomers) or a polyurethane.

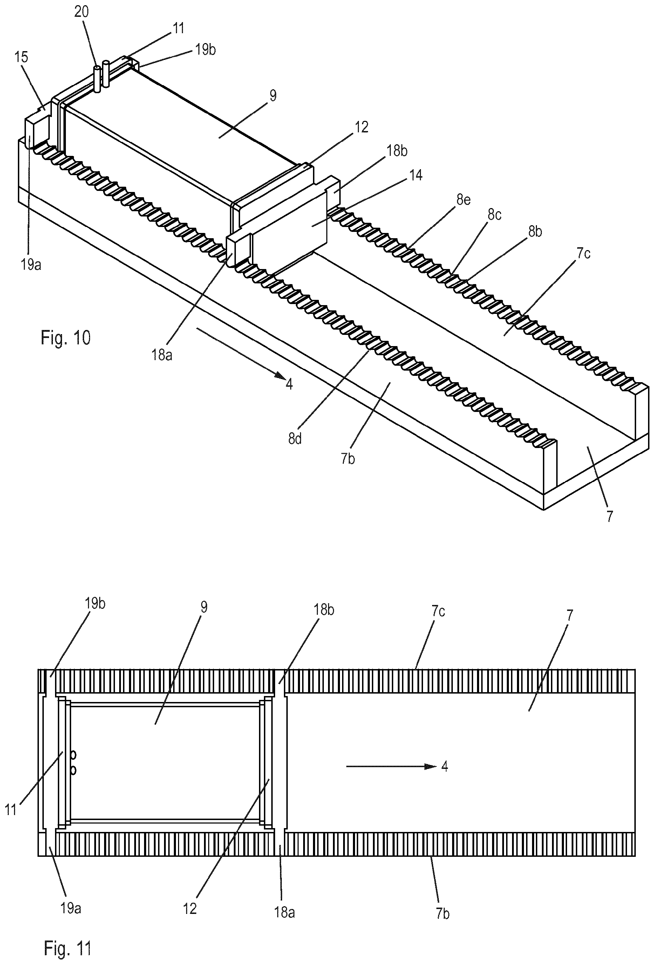

Applying an actuation voltage across each polymer layer using the conductive layers ensures that the polymer layer is extended in the lateral direction as its thickness is reduced. By applying the actuation voltage across all layers, the dimensions of the EAP actuator along the axis defined by the delivery direction 4 is reduced. When no voltage is applied, or when the electrodes are discharged, the EAP layers relax and elastically deform back to their original shape. Proximal and distal end plates 11,12 (Figure 2) are connected to the proximal and distal ends of the EAP actuator 9 such that modulation of the actuation voltage results in a reciprocating movement of the end plates relative to each other and the reciprocating movement may be used to advance the gear rack 7 as will be explained in the embodiment shown in Figures 2 to 9. The end plates are required for transferring the force from the linear EAP actuator 9 to an external part such as the gear rack 7. Contraction of the EAP actuator may also be used to advance ("crawl") the EAP actuator itself with its end plates versus a fixed gear rack as shown in the embodiment in Figures 10 and 11. The device 1 comprises a not shown external housing or a housing part such as a reservoir (cartridge) holder. The housing provides mechanical support to, amongst others, the EAP actuator and the reservoir. In the embodiment shown in Figures 2 to 9 one of the end plates 11,12 or parts that are coupled to one of the two end plates are coupled to the housing. In the embodiment presented in Figures 10 and 11, the gear rack 7 is coupled to the housing. In both embodiments, the reservoir 2 is axially fixed with respect to the housing by a reservoir holder.

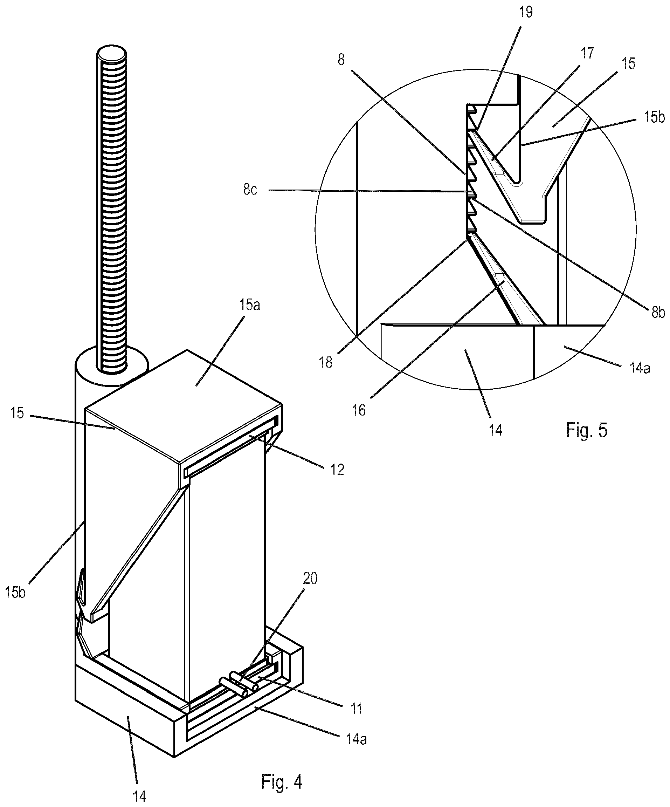

A drive mechanism for the device 1 for administering a fluid medicament with a moving gear rack 7 is presented in Figure 2 and 3. A retaining means 14 is directly or indirectly coupled to the housing and a drive means 15 is axially moveable with respect to the housing in a direction parallel to the delivery direction 4. Preferably the drive means 15 is splined or linearly guided by the housing or directly splined to the retaining means 14. The EAP actuator 9 is located at least partially between the drive means 15 and the retaining means 14. The drive means 15 has a top surface 15a which guides the end plate 12 of the actuator (Figure 4). The end plate 12 is preferably splined to the top surface 15a such that axial movements of the end plate 12 are transferred to the top surface 15a and to a connecting surface 15b of the driving means 15, the connecting surface 15b being oriented perpendicular to the top surface 15a. The retaining means 14 comprises a bottom surface 14a and a connecting surface 14b extending therefrom. The proximal end plate 11 of the EAP actuator is splined to the retaining means 14 to prevent relative axial movement along the delivery direction 4 but allow for sideways insertion of the EAP actuator 9 in the retaining means 14. The proximal end plate 11 is splined to the bottom surface 14a in the embodiment of Figure 4. Preferably the connecting surfaces 14b, 15b are parallel to another and have linear guiding means (not shown) for guiding the driving means 15 along the delivery direction 4.

A detail of the driving means 15 and retaining means 14 is shown in Figure 5. The retaining means 14 comprises a retaining tooth 18 at the end of a flexible retaining arm 16. The retaining arm 16 is operatively coupled to the housing and preferably connected to the bottom surface 14a of the retaining means 14, or the retaining arm 14a is part of the proximal end plate 11 of the EAP actuator 9. A driving arm 17 is operatively coupled to the driving means 15 such that the driving arm 17 is axially moveable with respect to the retaining arm 16. The driving means 15 comprises a driving tooth 19 at the end of the flexible drive arm 17. The drive arm 17 preferably extends from the connecting surface 15b of the driving means 15. The connecting surface 15b has a length to accommodate the EAP actuator 9, such that, in this particular example, the retaining tooth 18 and the driving tooth 19 are close together as each of them engages the gear rack 7. Alternatively, the driving tooth 19 is connected to the distal end plate 12 of the EAP actuator. The retaining tooth 18 engages a tooth of the series of serrated teeth 8 of the gear rack 7 and the driving tooth 19 engages a different tooth of the series of serrated teeth, e.g. the drive tooth and retaining tooth are axially spaced apart. Each tooth of the series of serrated teeth is oriented perpendicular to the delivery direction 4 and comprises a surface 8b that is essentially perpendicular to the delivery direction and an oblique surface 8c. The series of serrated teeth are preferably a series of asymmetric saw teeth. Alternatively, grooves or symmetric teeth are used. Both the retaining tooth 18 as well as the driving tooth 19 engage the surface 8b of the teeth on the gear rack 7 such that the gear rack is prevented from movement opposite to the delivery direction 4. The retaining means 14 is directly or indirectly coupled to the housing, such that the gear rack 7 is prevented from backward movement with respect to the housing or reservoir. When the EAP actuator 9 is activated by applying a voltage, then the distal end plate 12 and therewith the driving means 15 is moved from its original position to a retracted position and the driving tooth 19 is moved opposite (backward) to the delivery direction. The driving arm 17 slides across the oblique surface 8c of the tooth of the gear rack 7 and, due to the flexibility, the arm 17 flexes as the driving tooth jumps, or ratchets to the next tooth of the series of serrated teeth. During backward movement of the driving means 15, the gear rack 7 is held in its position by the retaining tooth 18 extending from and engaging another tooth of the gear rack. Both the retaining means 14 and the driving means 15 are part of one-way ratchets, a retaining ratchet formed by the retaining tooth 18 on the retaining arm 16 and a tooth of teeth 8 of the gear rack 7, and a driving ratchet formed by the driving tooth 19 at the end of driving arm 17 with another tooth of teeth 8 of the gear rack 7. When the actuation voltage for the EAP actuator 9 is reduced, the distal end plate 12 returns to its original position and the one-way driving ratchet slaves the gear rack 7 into the delivery direction 4 as the flexible retaining arm 16 flexes and the retaining tooth 18 jumps to the next tooth in the series of serrated teeth 8. By repeatedly applying a voltage on the EAP actuator 9, the driving means 15 is set in a reciprocating movement to forward the gear rack 7. Preferably, the stroke length of the reciprocating movement of the driving means 15 is at least equal, preferably above the distance between two teeth of the series of serrated teeth. The advancement of the gear rack 7 is used to advance the piston 3 in the cartridge 2 until the piston 3 reaches the narrowed section at the end of the cartridge. The force for advancing the piston 3 will increase and this may be measured by passive layers in the stack of EAP layers. Such a passive layer comprises two electrode layers and a dielectric layer there between which may be formed from the same polymer as the EAP layer. A sensor voltage is applied across the dielectric layer which is different from the actuation voltage of the EAP layer. Compression of the dielectric layer changes the distance between the passive electrodes and a change of capacitance is measured using the sensor voltage for detecting dimensional changes. The dimensional changes, for example the layer thickness, can be used to calculate the forces exerted onto the EAP actuator during advancement of the gear rack 7 when the mechanical properties of the dielectric layer are known, or when the sensor is calibrated in advance. Such sensor layers enable the detection of an empty cartridge or an occlusion in the fluid conduit between the cartridge and the patient.

In an alternative embodiment, the retaining arm 16 engaging the serrated teeth of the gear rack are replaced by another retaining means 14 based on a friction fit engagement with the gear rack. The friction fit engagement may be repeatedly released and established during advancement of the gear rack

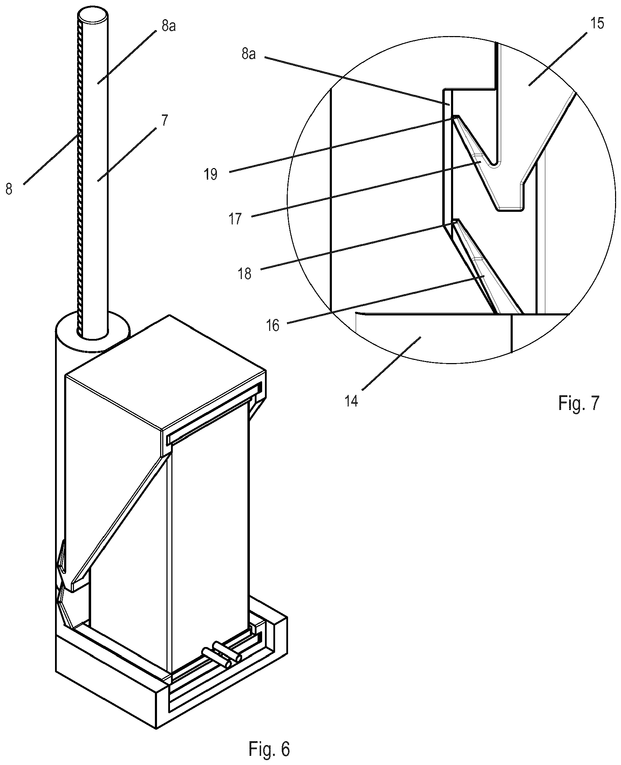

For a reusable administration device, the cartridge must be replaced when empty. For that purpose, the gear rack 7 is configured to be returned to the original position and an example for such reset mechanism is shown in Figures 6 and 7. The gear rack has been rotated around its longitudinal axis such that retaining tooth 18 and the driving tooth 19 are disengaged from the teeth 8 of the gear rack 7. The gear rack 7 may be pushed back to the original position before insertion of a full cartridge 2 into the housing of the device. Preferably, a cartridge holder comprising the empty cartridge is disengaged from the device 1, subsequently the gear rack 7 is rotated to disengage the driving and retaining ratchets for manual reset of the gear rack 7 to the starting position. Finally a new cartridge 2 is inserted into the cartridge holder which is re-attached to the housing.

In an alternative embodiment, the reset for the gear rack 7 is established by moving the retaining means 14 and driving means versus the gear rack such that the engagement with the gear rack is released for the reset. This may be combined with a gear rack having serrated teeth surrounding the gear rack. In the example described above, the gear rack has a smooth surface 8a, and this smooth surface will be replaced by serrated teeth as well.

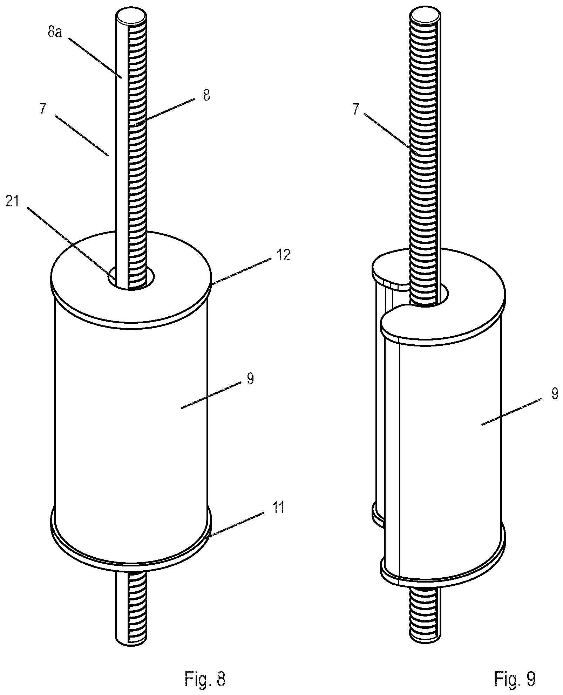

The electroactive polymer (EAP) actuator 9 comprises a stack of electroactive polymer layers, which are produced by a layer by layer stacking techniques, for example using printing technology. The layers may have a specific shape and therewith also a high degree of freedom for shaping the EAP actuator is feasible and two examples are shown in Figures 8 and 9. In Figure 8, the EAP actuator is cylindrically shaped having a central bore 21 in the EAP layers and end plates 11,12 for receiving the gear rack 7. The EAP actuator 9 surrounds the gear rack 7 leading to a space efficient design for the administration device 1. An EAP actuator with a C-shaped design for receiving the gear rack 7 is presented in Figure 9.

Another embodiment for a drive mechanism for the device 1 for administering a fluid medicament with a moving, or so-called crawling EAP actuator 9 versus a fixed gear rack 7 is presented in Figures 10 and 11. The retaining means 14 is connected to the distal end plate 12, e.g. the plate moving in the delivery direction 4 whereas the driving means 15 is connected to the proximal end plate 11 of the EAP actuator 9. The gear rack 7 is coupled to the housing or to the cartridge holder holding the cartridge. Thus a movement of the crawling EAP actuator 9 with respect to the gear rack 7 is used for advancing the piston 3 in the cartridge 2. The gear rack 7 comprises two separate gear racks 7b and 7c spaced apart perpendicular to the delivery direction 4 and each rack has a series of serrated teeth 8d, 8e. The series of serrated teeth 8d, 8e are axially displaced with respect to another along the delivery direction (Figure 11). The retaining means 14 comprises two retaining teeth 18a, 18b spaced apart perpendicular to the delivery direction that engage the two series of serrated teeth 8d, 8e, respectively. The driving means 15 comprises two drive teeth 19a, 19b spaced apart perpendicular to the delivery direction that engage the series of serrated teeth 8d, 8e, respectively. The drive teeth and retaining teeth form one-way driving ratchets and retaining ratchets with teeth of the gear rack, e.g. a surface 8b of the gear rack oriented perpendicular to the delivery direction engages a complementary perpendicular surface of the retaining teeth 18a, 18b or the driving teeth 19a, 19b whereas an oblique surface 8c of the gear rack engages complementary oblique surfaces the retaining teeth 18a,18b or the driving teeth 19a, 19b. When a voltage is applied across the EAP actuator 9, for example using connector 20, then the EAP actuator 9 is contracted and the driving means 15 moves into the delivery direction as the perpendicular surfaces 8b and complementary perpendicular surfaces of the retaining teeth 18a, 18b are in a form fit engagement with the gear rack preventing movement of the retaining means 14 in the delivery direction. The stroke length of the EAP actuator and the axial displacement of the two gear racks 7b, 7c is chosen such, that only one of the driving teeth 19a, 19b jumps to the next teeth in the series of serrated teeth 8d, 8e reforming the one way driving ratchet. When the voltage is reduced, the EAP actuator expands and one of the two retaining teeth 18a, 18b jumps to the next tooth in the series of serrated teeth 8d, 8e to reform the retaining ratchet as the reformed one-way driving ratchet prevents movement of the EAP actuator opposite to the delivery direction. When another (subsequent) actuation voltage is applied, the EAP actuator is contracted again and the other one of the driving teeth 19a, 19b jumps to the next teeth in the series of serrated teeth and when the voltage is reduced the EAP actuator expands and the other one of the retaining teeth 18a,18b jumps to the next tooth on the gear rack. Formation and release of the one way driving and retaining ratchet switches with each actuation stroke from one gear rack of the two racks 8d, 8e to the other one of the two gear racks 8d, 8e as the EAP actuator moves (or crawls) with respect to the gear rack in the delivery direction and this movement is used for advancing the piston in the reservoir.

In an alternative embodiment, the gear rack 7 comprises two separate gear racks 7b and 7c spaced apart perpendicular to the delivery direction 4 and each rack has a series of serrated teeth 8d, 8e. The series of serrated teeth 8d, 8e are not axially displaced with respect to another along the delivery direction, e.g the teeth 8d, 8e are aligned with another. When a voltage is applied, the EAP actuator contracts and both driving tooth 19a, 19b jump to the next tooth while the retaining tooth 18a,18b do not move on the gear rack. When the voltage is reduced, the EAP actuator expands and both driving tooth are in a fixed position whereas both retaining tooth 18a,18b jump to the next tooth in the series of teeth.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims.

### LIST OF REFERENCE SIGNS

| | | | |
|---|---|---|---|
| 1 | device for delivering a medicament | 12 | distal end plate |
| 2 | reservoir | 13 | liquid medicament |
| 3 | piston | 14 | retaining means |
| 4 | delivery direction | 14a | bottom surface |
| 5 | outlet | 14b | connecting surface |
| 6 | piercable septum | 15 | drive means |
| 7 | gear rack | 15a | top surface |
| 7a | distal end gear rack | 15b | connecting surface |
| 7b | gear rack | 16 | retaining arm |
| 7c | gear rack | 17 | drive arm |
| 8 | series of serrated teeth | 18 | retaining tooth |
| 8a | smooth surface | 18a | retaining tooth |
| 8b | perpendicular surface | 18b | retaining tooth |
| 8c | oblique surface | 19 | driving tooth |
| 8d | series of serrated teeth | 19a | driving tooth |
| 8e | series of serrated teeth | 19b | driving tooth |
| 9 | EAP actuator | 20 | connector |
| 11 | proximal end plate | 21 | bore |

## Claims

1. A device for delivering a fluid medicament comprising:
a) a reservoir with the fluid medicament,
b) a piston closing the reservoir and which can be moved with respect to the reservoir in a delivery direction towards an outlet of the reservoir for delivering the fluid medicament,
c) a gear rack for moving the piston in the delivery direction comprising a series of serrated teeth,
d) retaining means and driving means each engaging a tooth of the series of serrated teeth and each engagement prevents movement of the gear rack opposite to the delivery direction and allows movement of the gear rack in the delivery direction, and
e) wherein the retaining means is secured with respect to the reservoir, and
f) wherein the driving means can be set in a reciprocating motion relative to the retaining means into and opposite the delivery direction and the driving means slaves the gear rack to move together with the driving means into the delivery direction,
**characterized by** an electro-active polymer (EAP) actuator stacked between, and fixed to the retaining means and the driving means and which sets the driving means in the reciprocating movement.

2. A device for delivering a fluid medicament comprising:
a) a reservoir with the fluid medicament,
b) a piston closing the reservoir and which can be moved in a delivery direction towards an outlet of the reservoir for delivering the fluid medicament,
c) a gear rack comprising a series of serrated teeth, wherein the gear rack is secured with respect to the reservoir,
d) retaining means and driving means each engaging a tooth of the series of serrated teeth and each engagement prevents movement of the retaining means and driving means opposite to the delivery direction and allows movement of the retaining means and driving means with respect to the gear rack in the delivery direction,
wherein the driving means can be set in a reciprocating motion into and opposite the delivery direction and which is configured to slave the retaining means to move together with the driving means into the delivery direction to move the piston,
**characterized by** an electro-active polymer (EAP) actuator stacked between, and fixed to the retaining means and the driving means and which sets the driving means in the reciprocating motion.

3. The device according to claims 1 or 2 wherein the EAP actuator is a multilayered stack of capacitors comprising a dielectric layer and a conductor wherein the layers are oriented perpendicular to the delivery direction and wherein application of a voltage across the multilayered stack of capacitors results in a contraction or elongation along the delivery direction and wherein a voltage modulation, such as on/off switching, sets the driving means in the reciprocating motion.

4. The device according to claims 1 to 3 wherein the EAP actuator has an elongated shape with a disc shaped cross section having a central hole enclosing the gear rack along the delivery direction, or the EAP actuator has a C-shaped cross section and at least partially surrounds the gear rack along the delivery direction.

5. The device according to any of the previous claims wherein the multilayer stack of capacitors comprises a passive layer and wherein a sensor voltage is applied across the passive layers to measure a capacitance upon mechanical loading the EAP actuator to form a sensor layer measuring forces along the delivery direction.

6. The device according to any of the previous claims, wherein the retaining means comprises a retaining tooth located at a resilient retaining arm and wherein the retaining tooth forms a one-way retaining ratchet with a tooth of the series of serrated teeth of the gear rack.

7. The device according to any of the previous claims, wherein the driving means comprises a driving tooth located at a resilient driving arm and wherein the driving tooth forms a one-way driving ratchet with a tooth of the series of serrated teeth of the gear rack.

8. The device according to any of the previous claims wherein each tooth of the series of serrated teeth comprises a surface oriented essentially perpendicular to the delivery direction and a surface that is oblique to the delivery direction.

9. The device according to claim 8 wherein the retaining tooth and/or the driving tooth comprise a surface that is complementary to the oblique surface of the serrated teeth of the gear rack, and a surface that is complementary to the essentially perpendicular surface of the serrated teeth and wherein a form-fit engagement between the essentially perpendicular surfaces of the retaining tooth and the tooth of the gear rack prevent movement of the gear rack opposite to the delivery direction, and wherein a form-fit engagement between the essentially perpendicular surfaces of the driving tooth and the tooth of the gear rack slaves the gear rack in the delivery direction as the driving means is moved into the delivery direction.

10. The device according to claim 9 wherein the retaining arm of the retaining means is flexed as the gear rack is moved in the delivery direction and the complementary oblique surface of the retaining tooth slides over the oblique surface of a tooth of the series of tooth of the gear rack.

11. The device according to any of the previous wherein the gear rack comprises a plurality of series of serrated teeth that are axially displaced to another along the delivery axis and wherein each series of serrated teeth is each engaged with a retaining means and a driving means.

12. The device according to any of the previous claims wherein the gear rack can be rotated around the delivery direction to disengage the retaining means and/or the driving means from the gear rack such that the piston rod can be moved opposite to the delivery direction for resetting the gear rack to a starting position.

13. The device according to any of the previous claims wherein the movement of the driving means opposite to the delivery direction releases the engagement between the driving means and the series of serrated teeth while the engagement between the retaining means and the series of serrated teeth is maintained.

14. The device according to any of the previous claims wherein the movement of the driving means into the delivery direction releases the engagement between the retaining means and the series of serrated teeth of the gear rack while the gear rack is advanced into the delivery direction.

15. The device according to any of the previous claims wherein the device comprises a housing and wherein the housing or a part that is attached or attachable to the housing holds the reservoir, and wherein the retaining means is directly or indirectly fixed to the housing

16. An injection or infusion device comprising the device for delivering a fluid medicament according to any of the previous claims wherein the reservoir is a cartridge with the fluid medicament between the piston and a septum which is pierceable by a needle from an infusion set for delivery of the medicament..
